# EUROPEAN PATENT SPECIFICATION

(11) **EP 2 879 591 B1**
(45) Date of publication and mention of the grant of the patent: **28.03.2018**
(21) Application number: 13826022.9
(22) Date of filing: 03.08.2013
(51) Int. Cl.: A61B 17/04

(54) **SUTURE ANCHOR DEVICE**
NAHTANKERVORRICHTUNG
DISPOSITIF D'ANCRAGE DE SUTURE

(30) Priority: 03.08.2012 US 201213566845; 30.04.2013 US 201361817841 P
(43) Date of publication of application: 10.06.2015
(73) Proprietor: Stabilynx, Inc., Menlo Park CA 94025 (US)
(72) Inventor: DONAHUE, Joseph, P., Redwood City, CA 94063 (US); FEEZOR, Christopher, Redwood City, CA 94063 (US)
(74) Representative: Williams, Gareth Owen
(86) International application number: PCT/US2013/053524
(87) International publication number: WO 2014/022838

(56) References cited:
- EP-A1- 2 436 316
- EP-A1- 2 767 241
- EP-A2- 2 455 003
- WO-A2-02/21998
- WO-A2-2006/037131
- US-A- 5 534 011
- US-A1- 2003 065 361
- US-A1- 2011 238 111
- 'SMITHNEPHEW1. FOOTPRINT 123. Youtube.' FOOTPRINT 123 30 June 2011, XP054975861 Retrieved from the Internet: <URL:http://youtu.be/y2WCVa3GFcs> [retrieved on 2013-11-01]

## Description

### CROSS REFERENCE TO RELATED APPLICATIONS

This applications claims priority to U.S. App. No. 13/566,845, filed August 3, 2012, and claims priority to U.S. App. No. 61/ 817,841, filed April 30, 2013.

### FIELD OF THE INVENTION

The present invention generally relates to medical devices and to surgical implements. More particularly, the invention relates to suture anchor devices.

### BACKGROUND OF THE INVENTION

Soft tissue, such as tendons or ligaments, is typically displaced from its usual position in relation to the bone due to injury such as rupturing or tearing. Rotator cuffs, elbows, knees, ankles, and other joints are particularly prone to this type of injury. Injuries can be treated by attaching the soft tissue to the bone. Attaching soft tissue to bone often requires the use of suture anchors. Generally, a bone anchor with pre-loaded sutures is deployed into bone by inserting the anchor into an opening drilled into the bone. The pre-loaded sutures are used to attach the soft tissue to the bone by suture fixation techniques such as knot-tying, or by insertion of the suture into a knotless anchor for fixation.

Surgical anchor repairs suffer risk of biomechanical failure. Reported failures include suture cutting through bone tunnels, suture breakage, knot slippage, suture anchor pull out, and soft tissue failure at the suture-tendon junction. There is a need for a suture anchor device that will lower the risk of such biomechanical failure.

EP2455003 A2 discloses a suture anchor device for anchoring a suture to bone in which the suture anchor device is to be implanted, the suture anchor device comprising: a body including a bone engaging portion and a lumen; and a thread linked to the body by a first end and a second end of the thread engaging with the body, the thread being doubled and looped.

### BRIEF SUMMARY OF PREFERRED EMBODIMENTS OF THE INVENTION

The invention is directed to a suture anchor device according to claim 1. Preferred embodiments are recited in the dependent claims. The suture anchor device comprises a suture anchor with a thread-holding structure for holding sutures. A loading structure is threaded through the thread-holding structure. The loading structure has an aperture for being loaded with a suture after deployment of the suture anchor. When the aperture is pulled through the thread-holding structure, sutures loaded into the aperture are pulled through the thread-holding structure, reloading the suture anchor.

### BRIEF DESCRIPTION OF THE DRAWINGS

Preferred embodiments of the present invention are illustrated by way of example, and not by way of limitation, in the figures of the accompanying drawings and in which like reference numerals refer to similar elements and in which:
**FIG. 1** is a diagrammatic view of a suture anchor device not forming part of the invention.
**FIG. 1A** **-** **FIG. 1C** are cutaway diagrammatic views of an exploded portion of a suture anchor device not forming part of the invention.
**FIG. 2A** **-** **FIG. 2C** are cutaway diagrammatic views of an exploded portion of the suture anchor device shown in **FIG. 1****.**
FIG. 2D-2F are diagrammatic views of a suture anchor device constructed in accordance with an embodiment of the invention.
**FIG.** 3A - FIG. 3C are diagrammatic views of a cross-section of the suture anchor device
shown in **FIG. 1** as the suture anchor device is being operated to load a suture after deployment of the anchor into bone.
**FIG. 4** is a diagrammatic view of the suture anchor device shown in **FIG. 1** as used in one stage after deployment into bone.
**FIG. 5** is a diagrammatic view of the suture anchor device shown in **FIG. 1** as used in one stage after deployment into bone.
**FIG. 6** is a diagrammatic view of the suture anchor device shown in **FIG. 1** as used in one stage after deployment into bone.
**FIG. 7** is a diagrammatic view of the suture anchor device shown in **FIG. 1** as used with a particular suturing configuration and multiple suture anchor devices in an example of a double-row tendon repair.
**FIG. 8** is a diagrammatic view of the suture anchor device shown in **FIG. 1** as used with a particular suturing configuration and with locking knotless suture anchors in an example of a double-row tendon repair.
**FIG. 9** is a diagrammatic view of the suture anchor device shown in **FIG. 1** as used with a particular suturing configuration and multiple suture anchor devices in an example of a double-row tendon repair.
**FIG. 10** is a diagrammatic view of the suture anchor device shown in **FIG. 1** as used with a particular suturing configuration in an example of a single-row tendon repair.
**FIG. 11** is a diagrammatic view of the suture anchor device shown in **FIG. 1** as used with a particular suturing configuration in an example of a single-row tendon repair using three anchors.

### DETAILED DESCRIPTION OF PREFERRED EMBODIMENTS OF THE INVENTION

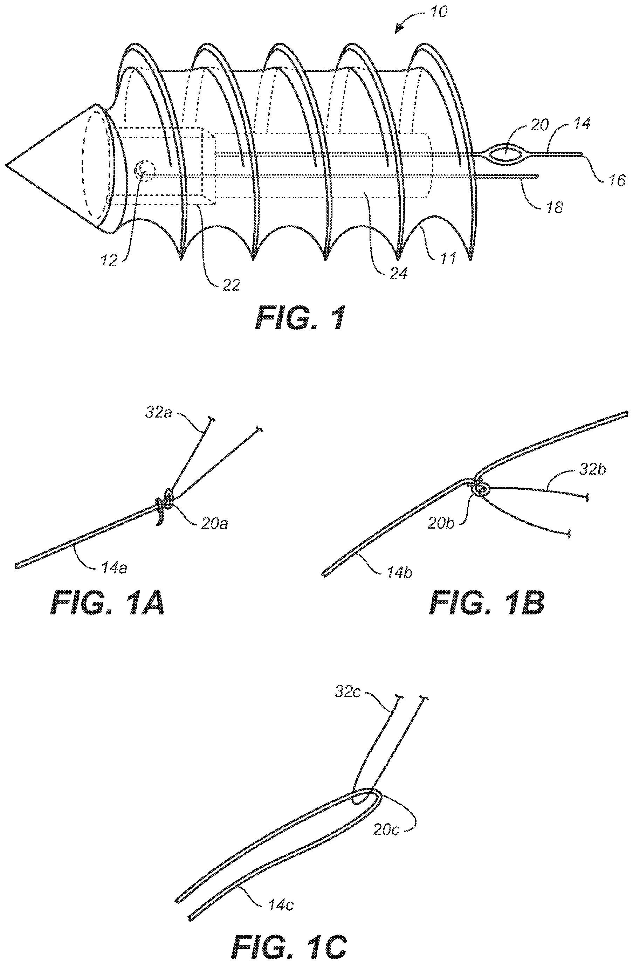

Referring to **FIG. 1****,** a suture anchor device 10 is shown not forming part of this invention. Suture anchor device 10 comprises a structure capable of loading a suture into the anchor after deployment of the anchor into bone. Suture anchor device 10 comprises eyelet 12, or other type of thread-holding member configured to receive or to be joined with sutures, filaments, wire, or other threadlike material. Suture anchor device 10 further comprises elongated loading member 14 having ends 16 and 18, and aperture 20 configured to be loaded or joined with a suture, filament, wire, or other threadlike material. In some embodiments, elongated loading member 14 is slippably threaded into eyelet 12. In some embodiments, aperture 20 is located at an end of loading member 14.

According to some embodiments, eyelet 12 of suture anchor device 10 is loaded with both loading member 14 and a suture before suture anchor device 10 is deployed into the bone. In some embodiments, loading member 14 is made of suture material and can be used as a standard suture with the anchor.

According to some embodiments, joining threadlike material to loading members 14 includes connecting threadlike member to loading member 14 in such a manner that causes threadlike material to travel and move with loading member 14 as loading member 14 is moved. For example, threadlike material is received into aperture 20. In another example, loading member 14 is tied to the threadlike material by forming a knot.

In one example, loading member 14 or the threadlike material are slippably threaded when loading member 14 or the threadlike material can be slipped out of position in the eyelet 12, in contrast with being fixed and unmovable.

While aperture 20 is shown in **FIG. 1** as formed from a split in the body of loading member 14, aperture 20 may be formed by other approaches, including those shown in **FIGS. 1A - 1C. FIG. 1A** illustrates portions of loading member 14a joined by knotting or other joining technique to form loop 20a at the end, which is configured to be loaded with or joined to threadlike material 32a. **FIG. 1B** illustrates portions of loading member 14b joined by knotting or other joining technique to form loop 20b at the middle, which is configured to be loaded with or joined to threadlike material 32b. In other examples, threadlike material 32 is joined to loading member 14 by tying loading member 14 around threadlike material 32. **FIG. 1C** illustrates loading member 14c bent to form open loop 2c, which is configured to be loaded or joined with threadlike material 32b. In one example of this configuration, the threadlike material forming loading member 14c is doubled and threaded into eyelet 12, with two ends of loading member 14c on one side of eyelet 12, and open loop 2c on the other side of 14c, extending through the inner lumen of the suture anchor.

In a preferred embodiment, eyelet 12 is located within cannulated suture anchor 11, at the deep end of the inner lumen of the suture anchor. Eyelet 12 is configured to receive and hold sutures, filaments, wire, or other threadlike material, such that the trailing ends of the threadlike material extend through inner lumen 24 of the suture anchor. In a preferred embodiment, eyelet 12 is formed as an aperture through tab structure 22 adjoining the suture anchor at the deep end of inner lumen 24.

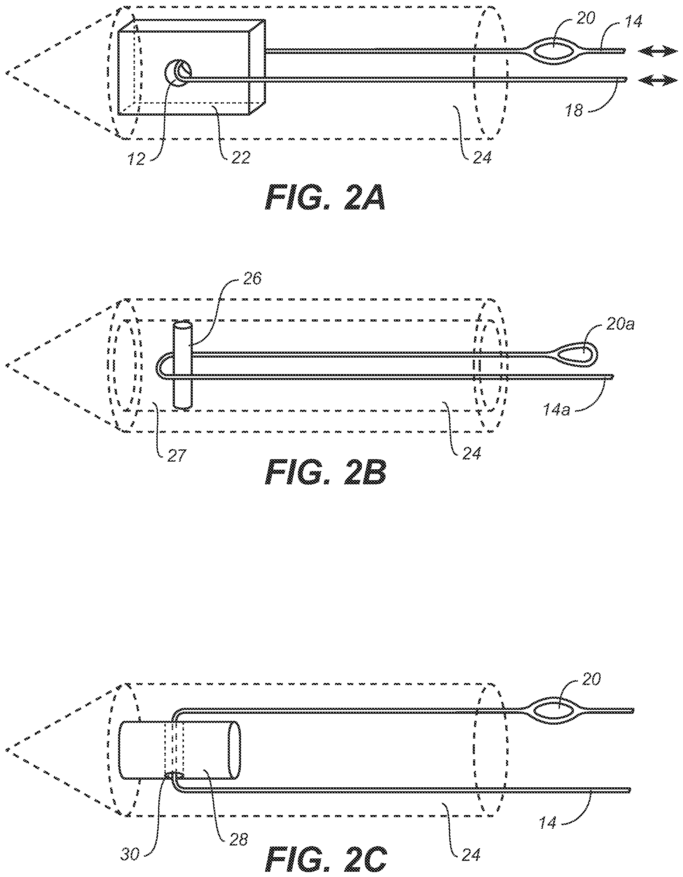

**FIG. 2A** **-** **FIG. 2C** are cutaway views of suture anchor device 10 that illustrate examples of configurations for a thread-holding member for suture anchor device 10 in embodiments not forming part of the invention. However, it is understood by those in the art that other variations of thread-holding members are capable of being used with suture anchor device 10.

**FIG. 2A** illustrates tab structure 22 as shown in **FIG. 1****.** In a preferred embodiment, eyelet 12, formed as an aperture through tab structure 22, is of a sufficient size to receive and hold multiple thicknesses or strands of threadlike material such that at least loading member 14 and a suture received through aperture 20 can slippably pass through the eyelet 12 within tab structure 22. In a preferred embodiment, tab structure 22 is of a size to allow lumen 24 to receive at once multiple thicknesses or strands of threadlike material.

**FIG. 2B** illustrates elongated barrier 26, including but not limited to a bar or pin, straight or having curves, within inner lumen 24 to form a thread-holding member 27 through which one or more threadlike members can be slippably threaded and held. In a preferred embodiment, as shown in **FIG. 2B****,** loading member 14a is loaded into the anchor through thread-holding member 27 and around elongated barrier 26. As shown, aperture 20a is at an end of loading member 14a. In a preferred embodiment, elongated barrier 26 is positioned within lumen 24 to allow lumen 24 to receive at once multiple thicknesses or strands of threadlike material into lumen 24, around elongated barrier 26, and out of lumen 24.

**FIG. 2C** illustrates peg structure 28, including but not limited to prisms of any polygonal base, such as a cylinder or quadrilateral prism. As shown, eyelet 30 is formed into peg structure 28 as a thread-holding member 30 through which one or more threadlike members, such as loading member 14, can be slippably threaded and held. In a preferred embodiment, the sizes of peg structure 28, eyelet 30, and lumen 24 are such that multiple thicknesses or strands of threadlike material are allowed to be received and held at once into lumen 24, through eyelet 30, and out of lumen 24.

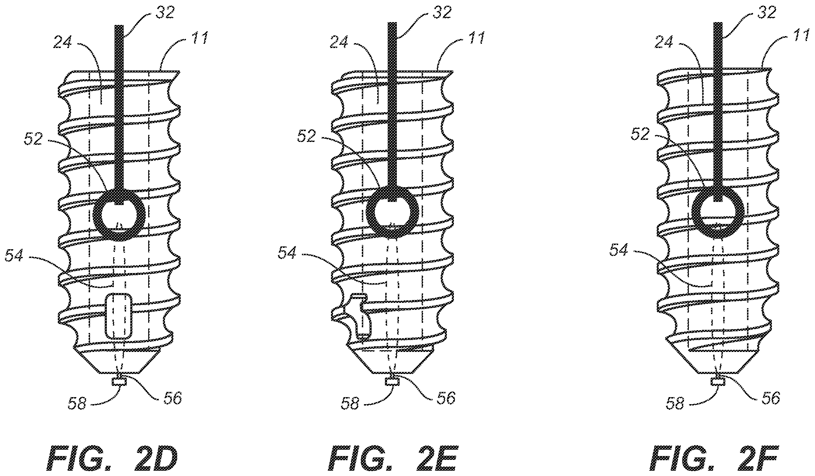

**FIG. 2D, FIG. 2E** and **FIG. 2F** are cutaway views of suture anchor device 10 that illustrate views of an example of a configuration for a free-swiveling thread-holding member 52 for suture anchor device 10 in embodiments of the invention. According to the embodiments, a thread-holding member 52, positioned within lumen 24 of suture anchor 11, is free to rotate or swivel with respect to the main body of suture anchor 11, the bone engaging portion of suture anchor device 10. The swiveling functionality decouples rotational orientation of the thread-holding member 52 from the rotational orientation of suture anchor 11, thereby allowing the orientation of threadlike member 32 that was threaded into thread-holding member 52 to be in a particular direction without regard to the rotation of the suture anchor device 10.

**FIG. 2D** illustrates a diagram of suture anchor 11 having a free-swiveling thread-holding member 52, according to embodiments of the invention. The view shown in **FIG. 2D** is of a first rotational orientation of suture anchor 11. Thread-holding member 52 is positioned within lumen 24 of suture anchor 11. As shown in **FIG. 2D****,** thread-holding member 52 is a ring that is linked to thread or suture 54. Thread or suture 54 is doubled and looped around thread-holding member 52, and passed through an aperture 56 of suture anchor 11, and terminated with a knot 58 or other structure for preventing the end of thread or suture 54 from passing through aperture 56. Thread-holding member 52 may be of a different structure or configuration than shown that allows free-swiveling movement relative to suture anchor 11 without departing from the invention. Tab structure 22, bar 26, and peg structure 28 as previously described may each be configured to be free-swiveling in accordance with embodiments of the invention.

**FIG. 2E** illustrates a diagram of the suture anchor 11 shown in **FIG. 2D****,** but instead rotated at a 45° angle about the screw axis of suture anchor 11 from the view shown in **FIG. 2D****.** As shown, while suture anchor 11 is rotated at a 45° angle, the angle of central structure of thread-holding member 52, suture 32, and thread or suture 54 is unchanged from the view in **FIG. 2D****.**

**FIG. 2F** illustrates a diagram of the suture anchor 11 shown in **FIG. 2D****,** but instead rotated at a 90° angle about the screw axis of suture anchor 11 from the view shown in **FIG. 2D****.** As shown, while suture anchor 11 is rotated at a 90° angle, the angle of central structure of thread-holding member 52, suture 32, and thread or suture 54 is unchanged from the view in **FIG. 2D****.**

The decoupling of the rotational orientation of thread-holding member 52 from suture anchor 11 provides advantages that would be appreciated by those of skill in the art. Thread-holding member 52 can rotationally orient in a direction that minimizes the tortuosity of the path traversed by threadlike members threaded into thread-holding member 52. In contrast, a suture or other threadlike member loaded into a fixed thread-holding member may become twisted when suture anchor device 10 is deployed and screwed into a bone. Reducing tortuosity reduces the amount of force required to load a suture into the device with a loading member 14. Swiveling also allows a suture threaded through thread-holding member 52 to slide with minimal tortuosity when tensioning a suture within a particular repair construct.

In other embodiments, the suture anchor does not have a cannulated structure or inner lumen. In such embodiments, a thread-holding member, for example, is formed in other configurations, such as an eyelet formed in a solid-body suture anchor for receiving multiple thicknesses or strands of threadlike material.

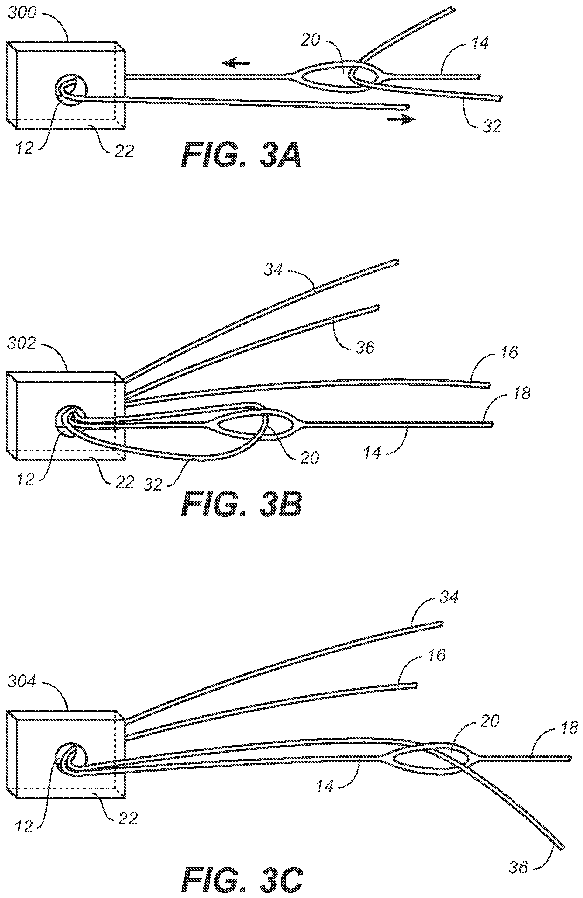

Stages in the operation of suture anchor device 10, according to embodiments not forming part of the invention, are shown in **FIG.** 3A - FIG.3C. Exploded portions of suture anchor device 10, comprising the thread-holding member within inner lumen 24, loading member 14, and suture 32, are shown in three stages. Stage 300 illustrates loading member 14 slippably threaded through eyelet 12 in tab structure 22. Suture 32 is received within aperture 20 of loading member 14. Arrows indicate the direction of travel of loading member 14 in embodiments of the invention for loading suture 32 into eyelet 12.

Stage 302 illustrates loading member 14 moved such that aperture 20 has been passed through eyelet 12 by pulling on end 18. Aperture 20 is shown as having been pulled into the near side of tab structure 22 as shown. At stage 302, suture 32 forms a loop and is doubled through eyelet 12. Ends 34 and 36 of suture 32 remain on the far side of tab structure 22 as shown. In embodiments of the invention where aperture 20 is at the end of loading member 14, no portion of loading member 14 remain on the far side of tab structure 22 after pulling aperture 20 through eyelet 12.

Stage 304 illustrates loading member 14 moved such that aperture 20 is further advanced through eyelet 12. By the movement of eyelet 12 while maintaining end 34 on the far side of the tab structure 22, end 36 of suture 32 is pulled through eyelet 12.

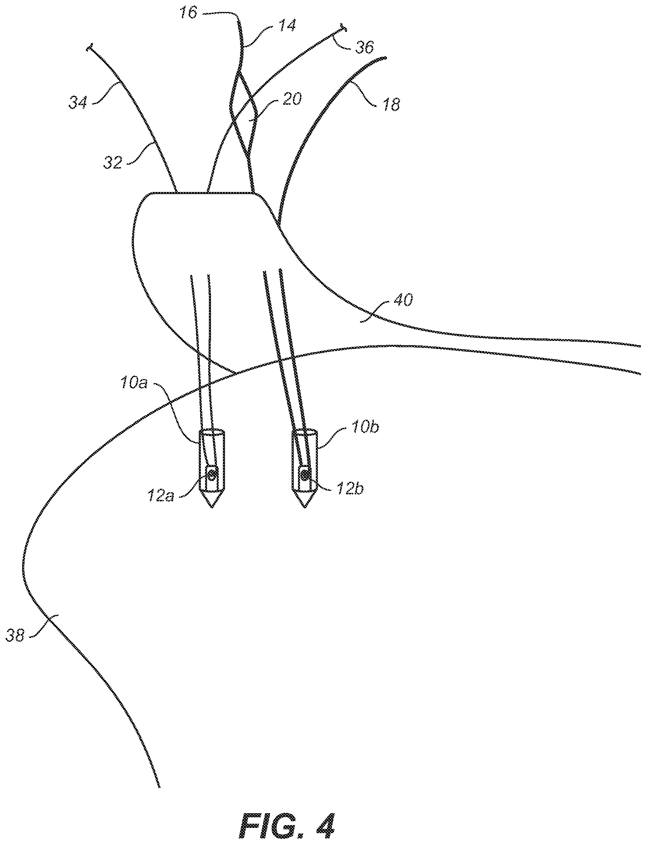

**FIGS. 4 - 6** illustrate stages of a method of using suture anchor device 10, according to embodiments not forming part of the invention. Because suture anchor device 10 is capable of loading sutures or other threadlike material through a suture anchor after deployment into bone, suture anchor device 10 allows for a new surgical technique according to embodiments of the invention for slippably threading a suture through thread-holding members of more than one suture anchor. Using this technique provides the advantage of increasing bony pull out strength, increasing soft tissue fixation, and optimizing the compression on the exposed bone surface to enhance biologic healing. This technique provides a further advantage in that unlike current methods of using knotless suture-locking anchors to adjoin a suture to two anchors, sutures linked by this technique using suture anchor device 10 remain slippably threaded into suture anchors instead of locked in place into a knotless anchor. Suture anchor device 10 linked by this technique also provides a further advantage of being used as a medial row anchor and a lateral row anchor.

**FIG. 4** illustrates a stage in the use of suture anchor 10 according to one embodiment not forming part of the invention. As shown, suture anchor devices 10a - 10b are deployed into bone 38 using approaches compatible with the type of suture anchor being used. In one example configuration, at the time of deployment, in suture anchor 10b, loading member 14 is slippably threaded into eyelet 12b, whereas in suture anchor 10a, loading member 14 is not in eyelet 12a, and suture 32 is in eyelet 12a. After deployment, in some embodiments, ends 16 and 18 of loading member 14, and ends 34 and 36 of suture 32, are passed through tendon 40, or other soft tissue, using suture passing techniques as available in the art. For example, as shown, ends 34 and 36 and ends 16 and 18 are passed from the articular side of the tendon to the bursal side of the tendon. End 36 of suture 32 is received through aperture 20 of loading member 14 on the bursal side of the tendon.

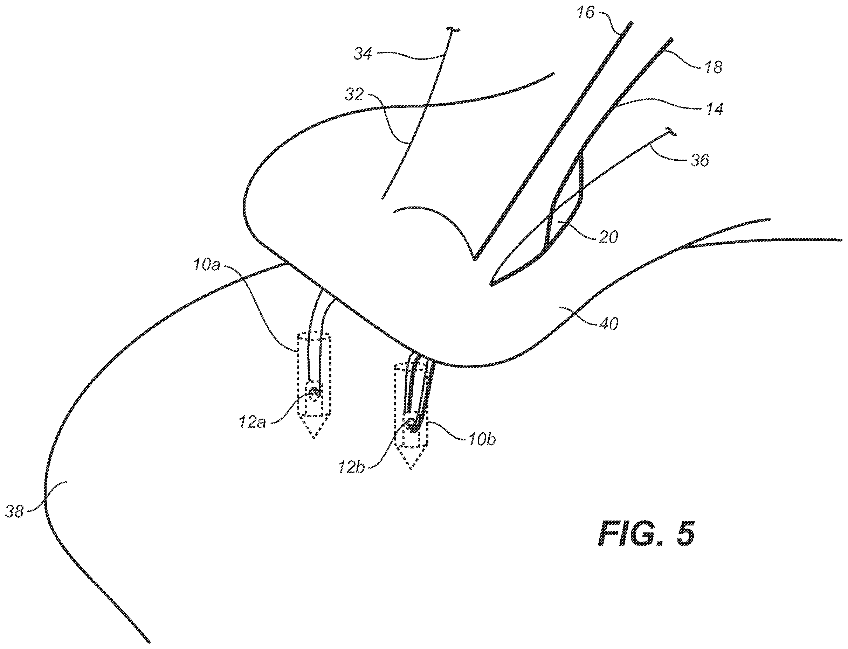

**FIG. 5** illustrates a stage in the use of suture anchor device 10 after the stage shown in **FIG. 4****,** in accordance with embodiments not forming part of the invention. End 18 of loading member 14 is pulled, passing a length of loading member 14 through eyelet 12. The suture received through aperture 20 travels with loading member 14 as it is pulled from the bursal side of the tendon to the articular side, and into the inner lumen of the suture anchor 11, and through eyelet 12. End 36 of suture 32 is pulled by aperture 20 through eyelet 12b, thereby loading suture 32 into eyelet 12b.

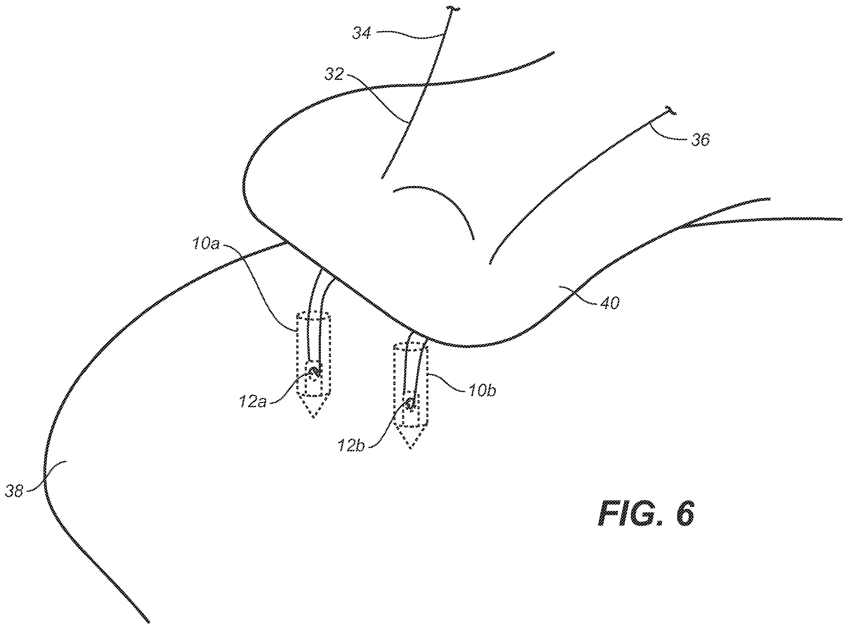

**FIG. 6** illustrates the final stage in the use of suture anchor device 10 for slippably linking at least two suture anchors in accordance with embodiments not forming part of the invention. As shown, loading member 14 has been removed from suture anchor device 10b. Suture 32 is slippably threaded through both eyelet 12a and eyelet 12b by use of loading member 14 in the technique described above. While the above description describes one sequence of steps for loading and passing suture 32 through anchors 10a and 10b and tendon 40, it is understood that other sequences of steps may be employed. For example, loading member 14 does not need to be passed through tendon 40 using conventional suture-passing techniques if suture 32 is passed using conventional techniques instead.

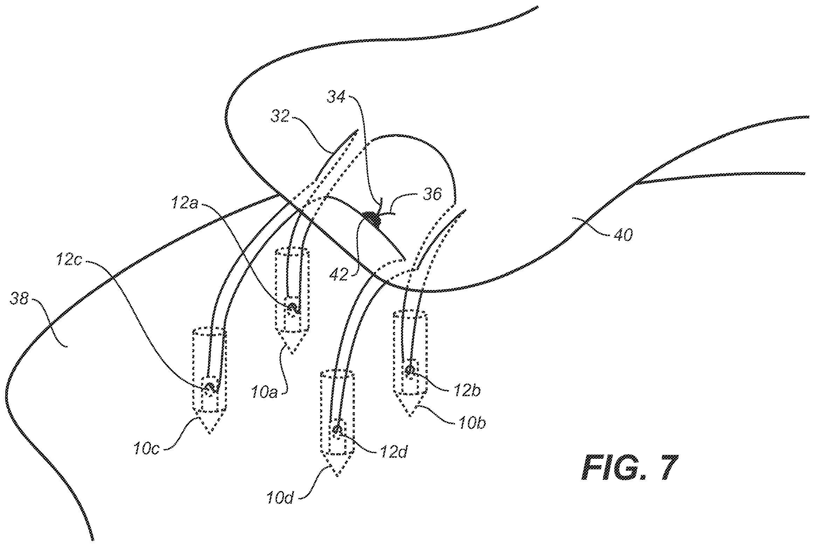

**FIG. 7** illustrates an example of using suture anchor device 10 in accordance to the principles of the embodiments not forming part of the invention described above. As shown, four suture anchors 10a - 10d are deployed into bone 38. Loading member 14 (not shown) of each of the four suture anchors 10a - 10d was used according to the methods and techniques discussed above to link suture 32 into each of the four suture anchors while being passed in and out of tendon 40. Ends 34 and 36 of suture 32 are pulled tight and tied into knot 42, while the length of suture 32 is passed through each of eyelets 12a - 12d, and remain slippably threaded therein. In this configuration, tendon 40 is compressed onto the bone between suture anchors 10a - 10d enhancing biologic healing and providing a watertight repair. Using this technique further provides the advantage of increasing bony pull out strength, and increasing soft tissue fixation.

While suture 32 is shown in **FIGS. 4 - 7** as the only threadlike member through eyelets 12a - 12d, it is understood by those skilled in the art that loading member 14 can be used to load multiple threadlike members through an eyelet as necessary for the surgical repair. For example, before loading member 14 is removed from an eyelet, aperture 20 is reloaded with another suture and loading member is pulled through the eyelet again.

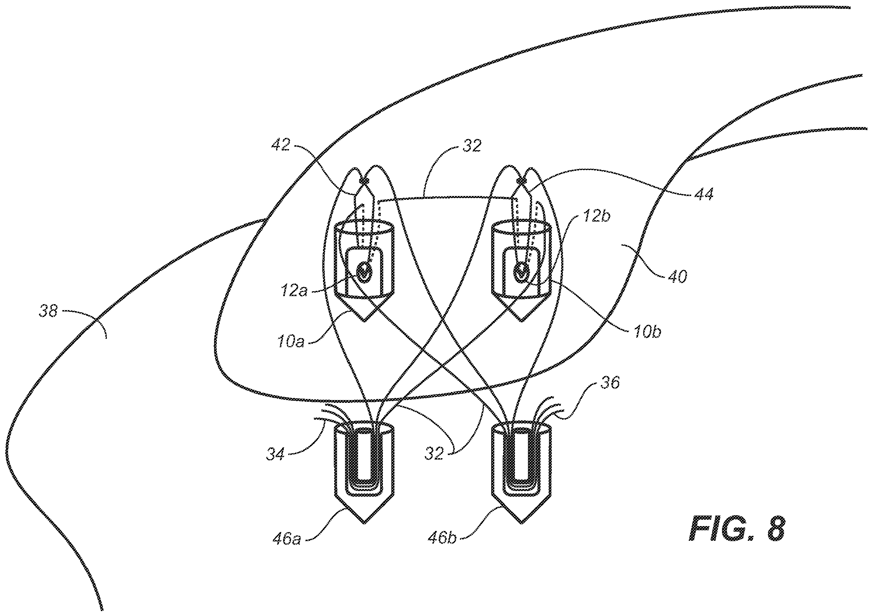

The techniques shown in **FIGS. 4 - 7** are able to be used with other suture patterns, as illustrated in **FIGS. 8 - 10****.** **FIG. 8** illustrates an example of one suturing configuration for using knotless locking anchors with the above-described techniques for passing suture 32 through anchors 10a - 10b using loading member 14 (not shown) in each of the anchors in a double-row repair. As shown, anchors 10a - 10b are deployed into the bone. Using techniques described above with reference to **FIGS. 4** and **5****,** suture 32 is threaded through anchors 10a and 10b using loading member 14 in each respective member, passing up, across, and down tendon 40 to compress tendon 40 to the bone. Each of anchors 10a and 10b is also threaded with sutures 42 and 44, which are passed through tendon 40 and tied as horizontal mattress sutures. Ends 34 and 36 of suture 32 are crossed over tendon 40. Suture ends of sutures 42 and 44 are also positioned over tendon in a crossed and square configuration. As shown, one end from each suture is gathered and fixed into locking knotless anchors 46a and 46b.

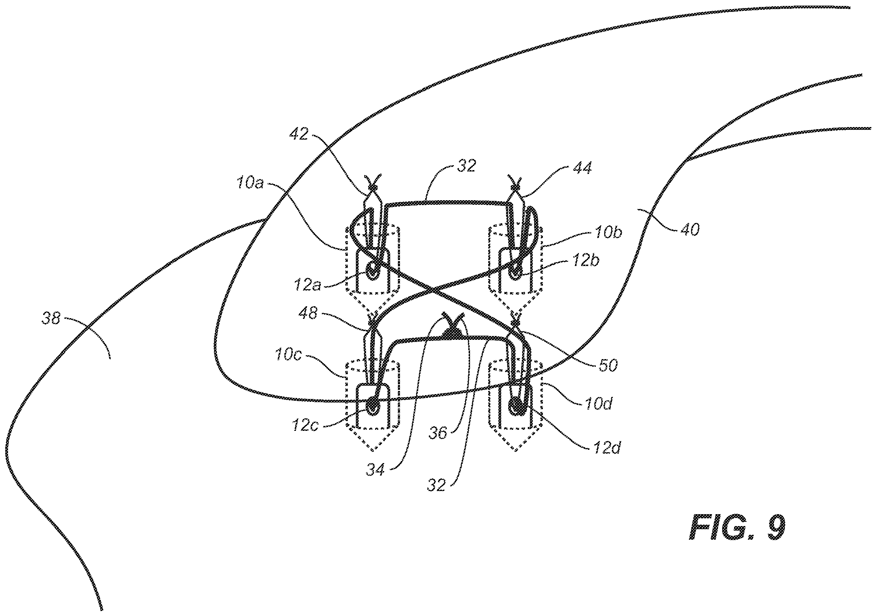

**FIG. 9** illustrates an example of one suturing configuration for using the above-described techniques for passing suture 32 through anchors 10a - 10d using loading member 14 (not shown) in each of the anchors in a double-row repair. As shown, anchors 10a - 10d are deployed into bone 38. Using the techniques shown described above with reference to **FIGS. 4** and **5****,** suture 32 is threaded through anchors 10a and 10b using loading member 14 in each respective anchor. Ends 34 and 36 of suture 32 are crossed over the top of tendon 40, are passed through tendon 40, and are threaded into anchors 10c and 10d respectively using loading member 14 in each respective anchor. Ends 34 and 36 are pulled to tension and compress tendon 40 against bone 38 and are tied into a knot, thereby linking anchors 10a - 10d. Each of anchors 10a - 10d is also threaded with sutures 42, 44, 48 and 50, passed through tendon 40 and tied as horizontal mattress sutures to provided added strength to the repair. In some embodiments, sutures 42, 44, 48 and 50 are threaded into anchors 10a - 10d before deployment into bone 38. In some embodiments, sutures 42, 44, 48 and 50 are threaded into anchors 10a - 10d after deployment into bone as needed.

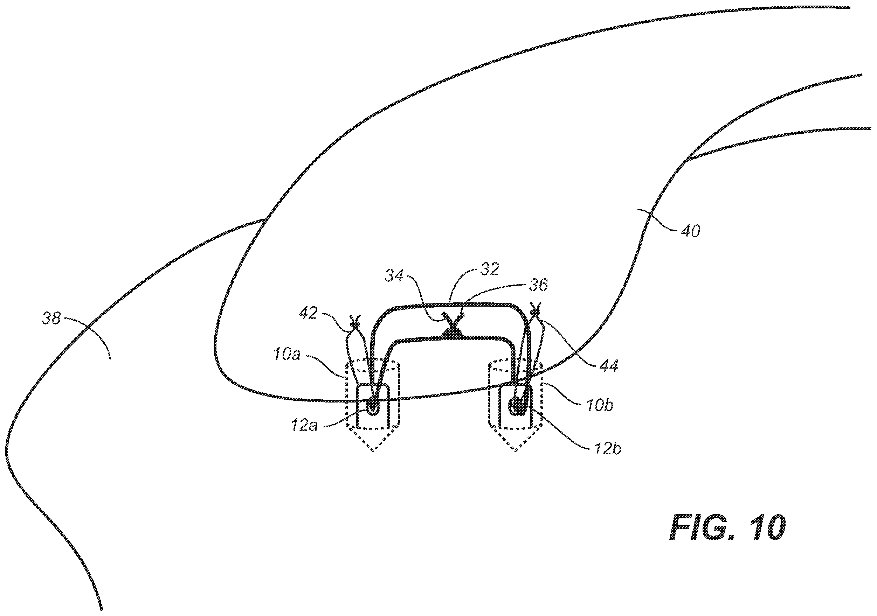

**FIG. 10** illustrates an example of one suturing configuration for using the above-described techniques for passing suture 32 through anchors 10a and 10b in a single-row repair. As shown, anchors 10a and 10b are deployed into the bone. Using techniques described above with reference to **FIGS. 4** **and** **5****,** suture 32 is threaded through anchors 10a and 10b using loading member 14 in each respective member, passing up, across, and down tendon 40 to compress tendon 40 to the bone. Each of anchors 10a and 10b is also threaded with sutures 42 and 44, which are passed through tendon 40 and tied as horizontal mattress sutures. Ends 34 and 36 of suture 32 are tied together to achieve the compression between the two anchors over tendon 40.

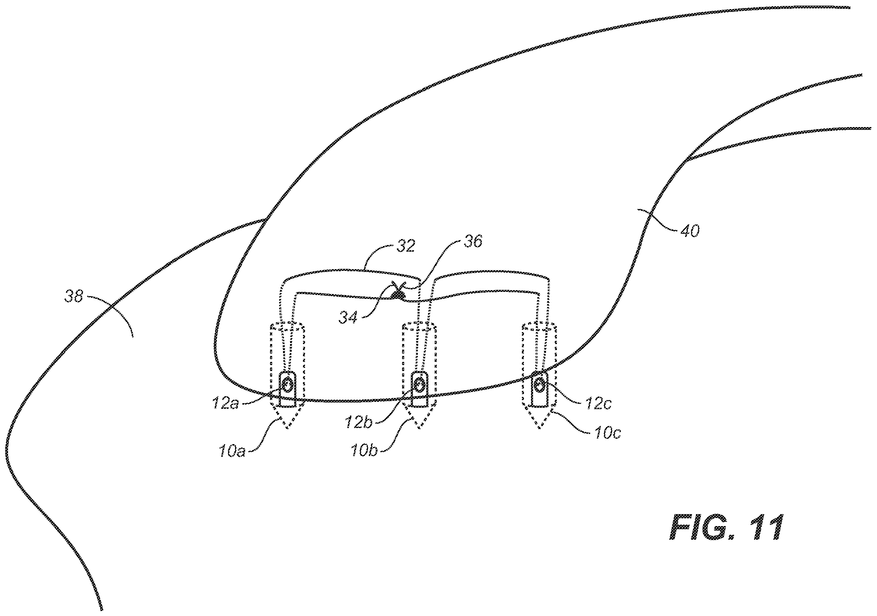

**FIG. 11** illustrates an example of one suturing configuration for using the above-described techniques for passing suture 32 through anchors 10a - 10c in a single-row repairing using three anchors. As shown, anchors 10a - 10c are deployed into the bone. Using techniques described above with reference to FIGS. 4 and 5, suture 32 is threaded through anchors 10a - 10c using loading member 14 in each respective member, passing up, across, and down tendon 40 to compress tendon 40 to the bone. Ends 34 and 36 of suture 32 are tied together to achieve the compression between the two anchors over tendon 40. In this configuration, tendon 40 is compressed onto the bone between suture anchors 10a - 10c enhancing biologic healing and providing a watertight repair. Using this technique further provides the advantage of increasing bony pull out strength, and increasing soft tissue fixation.

While **FIGS. 7** and **9 - 11** illustrate ends 34 and 36 tied together as a knot to complete the suture pattern, it is possible for ends 34 and 36 to be fixed by other surgical techniques. For example, a knotless locking anchor, in addition to the illustrated anchors, deployed into the bone can receive ends 34 and 36 for locking and fixing without tying any knots.

As described in some of the above embodiments, suture anchors are deployed into bone before a suture or other threadlike member is threaded into the suture anchor. However, it is understood that the suture anchor may be deployed into the bone at any reasonable point in the sequence of steps during surgery.

Other features, aspects and objects of the invention can be obtained from a review of the figures and the claims. It is to be understood that other embodiments of the invention can be developed and fall within the scope of the invention and claims.

The foregoing description of preferred embodiments of the present invention has been provided for the purposes of illustration and description. It is not intended to be exhaustive or to limit the invention to the precise forms disclosed. Various additions, deletions and modifications are contemplated as being within its scope. The scope of the invention is, therefore, indicated by the appended claims rather than the foregoing description. Further, all changes which may fall within the meaning and range of equivalency of the claims and elements and features thereof are to be embraced within their scope.

## Claims

1. A suture anchor device (10) for anchoring a suture (32) to bone in which the suture anchor device is to be implanted, the suture anchor device comprising:
a body (11) including a bone engaging portion and a lumen (24);
a thread (54); and
a free-swiveling thread-holding member (52) positioned within the lumen and secured to the body in a manner where the free-swiveling thread-holding member is free to rotate or swivel with respect to the body, the free-swiveling thread-holding member configured such that the suture can pass through the free-swiveling thread-holding member, wherein the thread (54) is linked to the body by a first end and a second end of the thread engaging with the body, the thread (54) doubled and looped around the thread-holding member (52) forming only one loop in the lumen of the body between the first end and the second end of the thread.

2. The suture anchor device of claim 1, wherein the free-swiveling thread-holding member includes a ring (52) through which the suture can pass.

3. The suture anchor device of claim 2, wherein the thread is looped around the ring.

4. The suture anchor device of claim 2, wherein the thread passes through an aperture (56) in the body in linking the ring to the body.

5. The suture anchor device of claim 1, wherein the free-swiveling thread-holding member includes a tab structure (22) including an eyelet (12) through which the suture can pass.

6. The suture anchor device of claim 1, wherein the free-swiveling thread-holding member includes a peg structure (28) including an eyelet (30) through which the suture can pass.

7. The suture anchor device of claim 1, wherein the first and second ends of the thread pass through an aperture (56) of the body.

## Patentansprüche

1. Nahtankervorrichtung (10) zum Verankern einer Naht (32) an einem Knochen, in welchem die Nahtankervorrichtung implantiert werden muss, wobei die Nahtankervorrichtung umfasst:
einen Körper (11), einschließend einen Knocheneingriffsabschnitt und ein Lumen (24);
einen Faden (54); und
ein freischwenkbares Fadenhalteglied (52), welches innerhalb des Lumens angeordnet ist und am Körper so befestigt ist, dass das freischwenkbare Fadenhalteglied sich gegenüber dem Körper frei drehen oder schwenken kann, wobei das freischwenkbare Fadenhalteglied so konfiguriert ist, dass die Naht durch das freischwenkbare Fadenhalteglied hindurchgehen kann, wobei der Faden (54) mit dem Körper durch ein erstes Ende und ein zweites Ende des Fadens verbunden ist, welche mit dem Körper in Eingriff stehen, wobei der Faden (54) verdoppelt und um das Fadenhalteglied (52) gewunden ist, wobei nur eine Schleife im Lumen des Körpers zwischen dem ersten Ende und dem zweiten Ende des Fadens gebildet wird.

2. Nahtankervorrichtung nach Anspruch 1, wobei das freischwenkbare Fadenhalteglied einen Ring (52) einschließt, durch welchen die Naht hindurchgehen kann.

3. Nahtankervorrichtung nach Anspruch 2, wobei der Faden um den Ring gewunden ist.

4. Nahtankervorrichtung nach Anspruch 2, wobei der Faden durch eine Öffnung (56) im Körper beim Verbinden des Rings mit dem Körper hindurchgeht.

5. Nahtankervorrichtung nach Anspruch 1, wobei das freischwenkbare Fadenhalteglied eine Zungenanordnung (22) einschließt, welche eine Öse (12) umfasst, durch welche die Naht durchgehen kann.

6. Nahtankervorrichtung nach Anspruch 1, wobei das freischwenkbare Fadenhalteglied eine Zapfenanordnung (28) einschließt, welche eine Öse (30) umfasst, durch welche die Naht durchgehen kann.

7. Nahtankervorrichtung nach Anspruch 1, wobei das erste und das zweite Ende des Fadens durch eine Öffnung (56) des Körpers hindurchgehen.

## Revendications

1. Dispositif d'ancrage de suture (10) pour ancrer une suture (32) sur l'os, le dispositif d'ancrage de suture devant être implanté, le dispositif d'ancrage de suture comprenant :
un corps (11) incluant une partie d'engagement de l'os et une lumière (24) ;
un fil (54) ; et
un élément de retenue de fil à pivotement libre (52) positionné dans la lumière et fixé sur le corps, d'une manière telle que l'élément de retenue du fil à pivotement libre peut tourner ou pivoter librement par rapport au corps, l'élément de retenue à pivotement libre étant configuré de sorte que la suture peut passer à travers l'élément de retenue du fil à pivotement libre, dans lequel le fil (54) est relié au corps par une première extrémité et et une deuxième extrémité du fil s'engageant dans le corps, le fil (54) étant doublé et enroulé en boucle autour de l'élément de retenue du fil (52) formant uniquement une boucle dans la lumière du corps entre la première extrémité et la deuxième extrémité du fil.

2. Dispositif d'ancrage de suture selon la revendication 1, dans lequel l'élément de retenue du fil à pivotement libre inclut une bague (52) à travers laquelle la suture peut passer.

3. Dispositif d'ancrage de suture selon la revendication 2, dans lequel le fil est enroulé en boucle autour de la bague.

4. Dispositif d'ancrage de suture selon la revendication 2, dans lequel le fil passe à travers une ouverture (56) dans le corps en reliant la bague au corps.

5. Dispositif d'ancrage de suture selon la revendication 1, dans lequel l'élément de retenue du fil à pivotement libre inclut une structure de patte (22) incluant un oeillet (30) à travers lequel la suture peut passer.

6. Dispositif d'ancrage de suture selon la revendication 1, dans lequel l'élément de retenue du fil à pivotement libre inclut une structure de cheville (28) incluant un oeillet (30) à travers lequel la suture peut passer.

7. Dispositif d'ancrage de suture selon la revendication 1, dans lequel les première et deuxième extrémités du fil passent à travers une ouverture (56) du corps.
